# EUROPÄISCHE PATENTANMELDUNG

(11) **EP 1 704 826 A1**
(43) Veröffentlichungstag der Anmeldung: **27.09.2006**
(21) Anmeldenummer: 06005137.2
(22) Anmeldetag: 14.03.2006
(51) Int. Cl.: A61B 19/00, A61B 5/103, A61F 2/30

(54) **Chirurgisches System zum Präparieren eines Implantats**

(30) Priorität: 23.03.2005 DE 102005014623
(71) Anmelder: Aesculap AG & Co. KG, 78532 Tuttlingen (DE)
(72) Erfinder: Giordano, Nicola, Dipl.-Ing. (FH), 78056 Villingen-Schwenningen (DE); Kienzle, Karl-Ernst, 78532 Tuttlingen (DE); Weiler, Andrea, Dr., 78570 Mühlheim (DE); Fink, Ulrich, Dr. Dipl.-Ing., 78532 Tuttlingen (DE); Jürgen, Fritz, Dr., 72144 Dusslingen (DE)
(74) Vertreter: Hoeger, Stellrecht & Partner Patentanwälte

(57) **Zusammenfassung**

Um ein chirurgisches System (10) zum Präparieren eines Implantats zum Ausfüllen eines aufgrund eines Traumas und/oder Degeneration entstandenen Defekts an einem Knochen und/oder einem Knorpel eines menschlichen oder tierischen Körpers, so zu verbessern, daß mit dem Implantat der Defekt möglichst paßformgenau ausgefüllt werden kann,
wird vorgeschlagen, daß mit dem System ein bereitgestellter Defektdatensatz verarbeitbar ist, welcher Daten insbesondere zur Beschreibung von Form, Fläche und/oder Volumen des Defekts enthält, und daß mit dem System aus mindestens einem bereitgestellten Implantatmaterial das dem Defekt in Form und Größe entsprechende Implantat unter Verwendung des Defektdatensatzes präparierbar ist.

## Beschreibung

Die vorliegende Erfindung betrifft ein chirurgisches System zum Präparieren eines Implantats zum Ausfüllen eines aufgrund eines Traumas und/oder Degeneration entstandenen Defekts an einem Knochen und/oder einem Knorpel eines menschlichen oder tierischen Körpers.

Ferner betrifft die vorliegende Erfindung ein Verfahren zum Präparieren eines Implantats zum Ausfüllen eines aufgrund eines Traumas und/oder Degeneration vorliegenden Defekts an einem Knochen und/oder einem Knorpel eines menschlichen oder tierischen Körpers.

Es ist bekannt, Knorpeldefekte an Gelenken, insbesondere traumatische Knorpeldefekte im Knie mit einer Fläche von 4 bis 15 cm², bevorzugt mittels einer biologischen Rekonstruktion zu versorgen. Eine mögliche Art der Versorgung besteht darin, insbesondere bei kleineren Defekten mit weniger als 4 cm² großen Defektflächen, den Defekt mit einem schwammartigen Trägermaterial auszufüllen. Vorzugsweise wird bei dieser Behandlungsmethode vor der Implantation, also vor dem Einsetzen eines das Trägermaterial umfassenden Ersatzkörpers, die subchondrale Platte des Knorpeldefekts gezielt etwas perforiert, was als "Mikrofrakturierung" bezeichnet wird. Infolge der Mikrofrakturierung der subchondralen Platte kommt es zum Einbluten autologer Knorpelzellen oder von Zellen aus dem Fettmarkgewebe des unter dem Defekt liegenden spongiösen Knochens in das Trägermaterial. Die Zellen aus dem Fettmarkgewebe können sich zumindest teilweise in Knorpelzellen differenzieren.

Eine alternatives Behandlungsverfahren bildet die autologe Chondrocyten Transplantation (ACT). Bei diesem Rekonstruktionsverfahren werden Knorpelzellen, vorzugsweise aus dem zu behandelnden Patienten entnommene, ex vivo kultiviert, vermehrt und anschließend in das schwammartige Trägermaterial, welches mindestens einen Teil eines in den Defekt einzusetzenden Ersatzkörpers bildet, eingebracht, was auch als "Beimpfen" des Trägermaterials mit den Knorpelzellen bezeichnet wird. Danach wird der das beimpfte Trägermaterial umfassende Ersatzkörper zur Ausfüllung des Defekts in den Körper des Patienten eingesetzt. Üblicherweise wird der das beimpfte Trägermaterial umfassende Ersatzkörper als "Transplantat" bezeichnet.

Nachfolgend wird die Bezeichnung "Implantat" sowohl für einen vor dem Einsetzen unbeimpftes Trägermaterial umfassenden Ersatzkörper, ein Implantat im eigentlichen Sinn, als auch für einen vor dem Einsetzen beimpftes Trägermaterial umfassenden Ersatzkörper ("Transplantat") verwendet.

Voraussetzung für beide Vorgehensweisen ist es, die Trägermaterialien intraoperativ optimal auf die individuelle Geometrie des tatsächlichen Defekts anzupassen. So ist insbesondere ein Spalt zwischen dem Trägermaterial und einer verbliebenen, gesunden Knorpelschulter keinesfalls akzeptabel. Zwar ist es bei einer offenen Operationstechnik einigermaßen gut möglich, Größe und Form des Defekts genau zu charakterisieren, dagegen ist die Bestimmung von Größe und Form des Defekts bei einer arthroskopischen Operationstechnik weitestgehend unmöglich.

Es ist daher Aufgabe der vorliegenden Erfindung, ein System und ein Verfahren der eingangs beschriebenen Art so zu verbessern, daß mit dem Implantat der Defekt möglichst paßformgenau ausgefüllt werden kann.

Diese Aufgabe wird bei einem chirurgischen System der eingangs beschriebenen Art erfindungsgemäß dadurch gelöst, daß mit dem System ein bereitgestellter Defektdatensatz verarbeitbar ist, welcher Daten insbesondere zur Beschreibung von Form, Fläche, Höhe und/oder Volumen des Defekts enthält, und daß mit dem System aus mindestens einem bereitgestellten Implantatmaterial das dem Defekt in Form und Größe entsprechende Implantat unter Verwendung des Defektdatensatzes präparierbar ist.

Mit einem solchen erfindungsgemäßen chirurgischen System läßt sich auf einfache Weise das Implantat präparieren. Alle hierfür notwendigen Informationen enthält der Defektdatensatz. Beispielsweise können mit dem System aus dem Defektdatensatz die Form des Defekts, dessen Fläche und/oder dessen Volumen ermittelt und weiterverarbeitet werden, um ein Implantat aus dem bereitgestellten Implantatmaterial zu präparieren, das in Form und Größe dem Defekt entspricht und diesen nach Implantation vollständig ausfüllt. Auf diese Weise kann eine Spaltbildung, beispielsweise zwischen einem Knorpelersatzimplantat und einer gesunden Knorpelschulter, vermieden werden. Derartige Spalte werden vom Körper nur sehr schwer regeneriert und können den Ausgangspunkt größer werdender Defekte bilden.

Vorteilhaft ist es, wenn das Implantat ein Knorpelersatzimplantat für die autologe Chondrocyten Transplantation (ACT) ist und wenn mit dem Knorpelersatzimplantat der einen Knorpeldefekt bildende Defekt ausfüllbar ist. Es ist also möglich, mit dem System ein Knorpelersatzimplantat zu präparieren, mit welchem ein Knorpeldefekt in einem menschlichen oder tierischen Körper behandelt werden kann.

Günstig ist es, wenn das Implantatmaterial ein für die Beimpfung mit Knorpelzellen geeignetes Trägermaterial ist, wenn das Knorpelersatzimplantat einen Träger für die Knorpelzellen umfaßt und wenn der Träger aus dem Trägermaterial präpariert ist. Ein derartiges Trägermaterial gestattet es, das Knorpelersatzimplantat vor der Implantation mit Knorpelzellen zu beimpfen und dann zu implantieren. Dadurch kann eine Regeneration des Knorpeldefekts deutlich verbessert werden.

Um das Beimpfen des Trägermaterials mit Knorpelzellen zu erleichtern, ist es vorteilhaft, wenn das Trägermaterial ein Vlies ist. Beispielsweise kann das Vlies ein Collagenvlies sein. Das Trägermaterial weist vorzugsweise eine Dicke auf, die einer Dicke oder Höhe des Defekts entspricht, beispielsweise bei einem Knorpeldefekt eine Dicke in einem Bereich von 1 bis 5 mm.

Grundsätzlich wäre es denkbar, das Knorpelersatzimplantat ohne Beimpfen mit Knorpelzellen zu implantieren, wie dies bereits eingangs beschrieben wurde. Günstigerweise ist der Träger jedoch mit Knorpelzellen beimpfbar. Auf diese Weise können Knorpelzellen gezielt in den Träger vor der Implantation eingebracht werden.

Gemäß einer bevorzugten Ausführungsform der Erfindung kann vorgesehen sein, daß der Träger mit Knorpelzellen beimpfbar ist, die im Labor vermehrte körpereigene Knorpelzellen sind, welche vor dem Einsetzen des Knorpelersatzimplantats aus dem menschlichen oder tierischen Körper entnommen wurden. Durch die Verwendung von körpereigenen Knorpelzellen kann eine Abstoßungsreaktion des Knorpelersatzimplantats minimiert oder sogar ganz vermieden werden.

Vorteilhaft ist es, wenn aus dem Defektdatensatz das Defektvolumen des Knorpeldefekts bestimmbar ist und wenn ein bereitgestelltes Implantationsvolumen von Knorpelzellen ausreicht, um den Träger für eine Implantation des Knorpelersatzimplantats ausreichend mit Knorpelzellen zu beimpfen. Wenn das Defektvolumen bekannt ist, kann die Vermehrung von Knorpelzellen optimiert werden, da nicht mehr Knorpelzellen gezüchtet werden müssen, als zum Ausfüllen des Defektvolumens erforderlich sind. Damit ist auch eine obere Grenze für ein bereitzustellendes Implantationsvolumen angebbar.

Vorteilhaft ist es, wenn das bereitgestellte Trägermaterial Hohlräume zur Aufnahme von Knorpelzellen aufweist, wenn die Hohlräume des Trägers ein Hohlraumvolumen aufweisen und wenn ein bereitgestelltes Implantationsvolumen dem Hohlraumvolumen oder in etwa dem Hohlraumvolumen des Trägers entspricht. Die Vermehrung der Knorpelzellen kann dadurch weiter optimiert werden, da in der Regel das Hohlraumvolumen des Trägers kleiner als das Defektvolumens ist. Dadurch kann die kostenintensive Vermehrung von Knorpelzellen auf das erforderliche Maß beschränkt werden.

Gemäß einer bevorzugten Ausführungsform der Erfindung kann eine Datenverabeitungseinrichtung vorgesehen sein zum Verarbeiten des Defektdatensatzes mit der Datenverarbeitungseinrichtung zu einem Präparierdatensatz, wobei der Präparierdatensatz Daten insbesondere zur Beschreibung von Form, Fläche, Höhe und/oder Volumen des zu präparierenden Implantats enthält. Der Präparierdatensatz kann zum Beispiel verwendet werden, um mit einem Bearbeitungswerkzeug oder einer Bearbeitungsvorrichtung das Implantatmaterial in gewünschter Weise zu bearbeiten, also ein dem Defekt entsprechendes Implantat auszubilden beziehungsweise zu präparieren.

Günstig ist es, wenn ein chirurgisches Instrument vorgesehen ist zum Eröffnen eines minimalinvasiven Zugangs in den menschlichen oder tierischen Körper zum Bestimmen des Defektdatensatzes. Mit dem erfindungsgemäßen System ist es daher nicht mehr erforderlich, beispielsweise ein Gelenk vollständig zu eröffnen, sondern es kann der Defektdatensatz unter Verwendung eines minimalinvasiven oder arthroskopischen Zugangs bestimmt werden.

Wie eingangs erwähnt, ist die Bestimmung der Defektgröße und damit des Defektdatensatzes relativ einfach, wenn eine offene Operationstechnik angewandt wird. Da beispielsweise bei der autologen Chondrocyten Transplantation Knorpelzellen arthroskopisch entnommen werden, wäre es wünschenswert, den Defektdatensatz, insbesondere dabei, auch arthroskopisch zu bestimmen. Studien haben gezeigt, daß jedoch selbst erfahrene Arthroskopeure durch die Verzerrung des Bildes bei der Arthroskopie, selbst bei der Verwendung von Tasthaken mit Meßfunktion, die Defektfläche um bis zu einem Faktor 2 falsch einschätzen. Daher ist es vorteilhaft, wenn ein Navigationssystem mit einer Nachweisvorrichtung vorgesehen ist, daß ein Meßinstrument vorgesehen ist, an welchem ein von der Nachweisvorrichtung des Navigationssystems detektierbares Referenzelement anordenbar ist zum Bestimmen von Lage und Orientierung des Meßinstruments im Raum und daß mit dem Meßinstruments der Defekt zum Bestimmen des Defektdatensatzes ausmeßbar und gleichzeitig mit dem Navigationssystem die Lage und Orientierung des Meßinstruments im Raum bestimmbar sind. Zur Bestimmung des Meßdatensatzes muß ein Operateur nichts mehr abschätzen, sondern kann mit Hilfe des Meßinstruments den Defekt hochpräzise ausmessen. Dies wird durch die Navigation des Meßinstruments möglich, da auf diese Weise Lage und Orientierung desselben während der Messung stets genau bekannt sind. Auch ein eventuell vom Arthroskopeur wahrgenommenes, verzerrtes Bild des Defekts wird somit bedeutungslos, da der Defektdatensatz die tatsächlichen geometrischen Daten des Defekts enthält.

Günstig ist es, wenn das Meßinstrument durch einen minimalinvasiven Zugang in den menschlichen oder tierischen Körper einführbar ist. Dadurch ist es nicht mehr erforderlich, eine offene Operationstechnik anzuwenden, sondern es kann bereits nach Eröffnung eines minimal invasiven Zugangs, beispielsweise zur Entnahme von körpereigenen Knorpelzellen, das Meßinstrument in den Körper eingeführt werden. Hierzu ist es günstigerweise in Form eines endoskopischen Instruments ausgebildet.

Gemäß einer bevorzugten Ausführungsform der Erfindung kann vorgesehen sein, daß das Meßinstrument eine Tastspitze aufweist und daß zum Ausmessen des Defekts ein Rand oder eine Begrenzung mit der Tastspitze palpierbar ist. Ein Operateur kann mit der Tastspitze den Rand oder die Begrenzung des Defekts abtasten, wobei gleichzeitig Positionsdaten der Tastspitze durch die Nachweisvorrichtung ermittelt werden. Aus dem auf diese Weise bestimmten Defektdatensatz kann dann zum Beispiel die Form, die Größe und/oder das Volumen des Defekts bestimmt werden.

Günstig ist es, wenn das Meßinstrument derart ausgebildet ist, daß der Defekt berührungslos ausmeßbar ist. Dies hat den Vorteil, daß der Defekt durch das Ausmessen nicht weiter vergrößert wird. Beispielsweise kann das Meßinstrument elektromagnetische Strahlung, insbesondere sichtbares Licht, oder Ultraschall zum Ausmessen des Defekts nutzen.

Gemäß einer weiteren bevorzugten Ausführungsform der Erfindung kann vorgesehen sein, daß das Meßinstrument eine optische Bilderfassungseinheit aufweist und daß mit der Bilderfassungseinheit mindestens ein Defektbild des Defekts aufnehmbar ist. Aus dem navigiert aufgenommenen Defektbild lassen sich alle für den Defekt relevanten Daten ermitteln, das heißt insbesondere Form, Fläche, Höhe und/oder Volumen des Defekts. Ferner hat die Bilderfassungseinheit den Vorteil, daß der Defekt berührungslos ausgemessen werden kann. Wenn ferner der Körper des Patienten auch navigiert wird, können so reale Raumdaten des Defekts über die optische Bildeinheit, auch in Echtzeit, erfaßt und mit einer Datenverarbeitungsvorrichtung weiterverarbeitet werden. Außerdem kann so insbesondere auch festgestellt werden, ob der Defekt nicht nur den Knorpel, sondern auch die darunterliegende subchondrale Platte oder sogar den Knochen betrifft. Ist dies der Fall, dann kann vor dem Einsetzen des Implantats der Defekt an der subchondralen Platte und/oder am Knochen durch Knochenspäne oder Knochenersatzmaterial aufgefüttert werden, um den Erfolg der Behandlung des Knorpeldefekts nicht zu gefährden.

Günstig ist es, wenn von der Bilderfassungseinheit ein Bilddatensatz des mindestens einen Defektbildes bereitstellbar ist und wenn der Bilddatensatz zum Defektdatensatz verarbeitbar ist. Vorzugsweise ist eine Datenverarbeitungseinrichtung vorgesehen zum Verarbeiten des Bilddatensatzes zum Defektdatensatz. Beispielsweise ist es so möglich, den Defektdatensatz zu erzeugen und, falls gewünscht und erforderlich, den Defektdatensatz zeitnah direkt zu verwenden, um während des chirurgischen Eingriffs das Implantat zu präparieren.

Damit auch ohne Zusatzbeleuchtung der Defekt ausmeßbar ist, ist es vorteilhaft, wenn als Bilderfassungseinheit mindestens ein Infrarotsensor vorgesehen ist. Mit dem mindestens einen Infrarotsensor läßt sich der Defekt berührungslos erfassen und ein Bilddatensatz ermitteln.

Bei einer bevorzugten Ausführungsform der Erfindung kann ein Bearbeitungswerkzeug zum Bearbeiten des Implantatmaterials vorgesehen und das Implantatmaterial mit dem Bearbeitungswerkzeugs unter Verwendung des Defektdatensatzes bearbeitbar sein. Es ist auf diese Weise möglich, mit dem Bearbeitungswerkzeug das Implantat in gewünschter Weise zu präparieren, da alle für die Präparierung des Implantats erforderlichen Daten im Defektdatensatz enthalten sind, insbesondere Form Größe, Fläche, Höhe und/oder Volumen des Defektes.

Grundsätzlich wäre es denkbar, daß das Bearbeitungswerkzeug Teil einer Bearbeitungsvorrichtung ist, welche nach Vorgabe des Defektdatensatzes gesteuert das Implantat präpariert. Vorteilhaft ist es jedoch, wenn das Bearbeitungswerkzeug ein von der Nachweisvorrichtung detektierbares Referenzelement aufweist und wenn das Implantatmaterial mit dem Bearbeitungswerkzeug unter gleichzeitiger Detektion von Lage und Orientierung des Bearbeitungswerkzeugs bearbeitbar ist. Man kann mit einem derartigen Bearbeitungswerkzeug unter Verwendung des Defektdatensatzes manuell das Implantat präparieren, zum Beispiel durch Aufzeichnen einer Kontur auf ein Implantatmaterial oder durch Ausschneiden mit einem Schneidwerkzeug, insbesondere mit einer Schere, einer Stanze oder einem Laser. Beispielsweise kann hierfür eine mit dem Bearbeitungswerkzeug abzufahrende Bewegungsbahn, also eine Sollkurve, an einer Anzeigevorrichtung, beispielsweise einem Bildschirm, angezeigt werden. Gleichzeitig kann eine Ist-Bewegungsbahn des Bearbeitungswerkzeugs der Sollkurve überlagert werden, so daß eine Bedienperson stets genau weiß, ob sie das Implantatmaterial in gewünschter Weise bearbeitet.

Günstig ist es, wenn das Bearbeitungswerkzeug ein Markierwerkzeug umfaßt zum Übertragen des Defektdatensatzes auf das Implantatmaterial. Beispielsweise läßt sich so das Implantatmaterial in gewünschter Weise kennzeichnen, um es anschließend zu bearbeiten. Inbesondere kann ein Trägermaterial, zum Beispiel ein Vlies, auf diese Weise entsprechend einer aus dem Defektdatensatz bekannten Fläche des Defektes markiert und nach dem Markieren ausgeschnitten werden.

Ferner kann es vorteilhaft sein, wenn das Bearbeitungswerkzeug eine Schneidvorrichtung umfaßt zum Ausschneiden des Trägers aus dem Implantatmaterial unter Verwendung des Defektdatensatzes. Mit dem Bearbeitungswerkzeug kann zum Beispiel entweder der Defektdatensatz auf das Implantatmaterial übertragen werden oder aber auch direkt ausgeschnitten werden mit der Schneidvorrichtung.

Um das Implantat vollautomatisch präparieren zu können, ist es günstig, wenn eine Bearbeitungsvorrichtung zum Bearbeiten des Implantatmaterials vorgesehen ist, wenn der Defektdatensatz auf die Bearbeitungsvorrichtung übertragbar ist und wenn das Implantatmaterial mit der Bearbeitungsvorrichtung bearbeitbar ist. Die Bearbeitungsvorrichtung kann unter Verwendung des Defektdatensatzes angesteuert werden, alternativ wäre es auch denkbar, daß die Bearbeitungsvorrichtung zusätzlich ein Referenzelement trägt, welches mit dem Navigationssystem überwacht werden kann, und auf diese Weise eine Bearbeitung des Implantatmaterials navigiert erfolgt.

Gemäß einer bevorzugten Ausführungsform der Erfindung kann ferner vorgesehen sein, daß die Bearbeitungsvorrichtung das Bearbeitungswerkzeug umfaßt. Es können für unterschiedliche Implantatmaterialien unterschiedliche Bearbeitungswerkzeuge vorgesehen sein, beispielsweise Stanz- oder Schneidewerkzeuge, insbesondere messerähnliche, oder auch Strahlschneidwerkzeuge wie Laser oder dergleichen.

Ferner kann vorgesehen sein, daß das System das Implantat umfaßt. Das Implantat ist damit Teil des Systems zur Behandlung eines Defekts an einem menschlichen oder tierischen Körper.

Die eingangs gestellte Aufgabe wird bei einem Verfahren der eingangs beschriebenen Art erfindungsgemäß dadurch gelöst, daß es die Schritte umfaßt:
- Bereitstellen mindestens eines Implantatmaterials,
- Bereitstellen eines Defektdatensatzes, welcher Daten zur Beschreibung insbesondere von Form, Fläche, Höhe und/oder Volumen des Defekts enthält, und
- Präparieren des den Defekt in Form und Größe entsprechenden Implantats aus dem Implantatmaterial unter Verwendung des Defektdatensatzes.

Mit dem erfindungsgemäßen Verfahren kann auf einfache Weise ein Implantat präpariert werden, mit dem der Defekt mindestens teilweise, vorzugsweise vollständig ausgefüllt werden kann. Zudem muß das Verfahren nicht von einem Arzt durchgeführt werden, denn durch das Bereitstellen des Defektdatensatzes kann das Implantat unabhängig von einem chirurgischen Eingriff präpariert und zum Einsetzen in den menschlichen oder tierischen Körper bereitgestellt werden.

Besonders vorteilhaft ist es, wenn der Defekt ein Knorpeldefekt ist, wenn das Implantat ein Knorpelersatzimplantat für die autologe Chondrocyten Transplantation (ACT) und wenn mit dem Knorpelersatzimplantat der Knorpeldefekt ausgefüllt wird. Mit dem erfindungsgemäßen Verfahren kann auf einfache Weise ein Knorpelersatzimplantat zur Behandlung beispielsweise eines Knorpeldefekts an einem Kniegelenk präpariert werden. Im Gegensatz zu bekannten Operationstechniken ist es nunmehr möglich, ein Knorpelersatzimplantat paßformgenau zu präparieren, sodaß kein Spalt zwischen dem implantierten Knorpelersatzimplantat und einem verbliebenen Knorpelrand entstehen kann.

Günstig ist es, wenn als Implantatmaterial ein für die Beimpfung mit Knorpelzellen geeignetes Trägermaterial bereitgestellt wird, wenn das Knorpelersatzimplantat einen Träger für die Knorpelzellen umfaßt und wenn der Träger aus dem Trägermaterial präpariert wird. Es ist auf diese Weise möglich, insbesondere körpereigene Knorpelzellen in ein Trägermaterial einzubringen und für die Implantation bereitzustellen. Derartige Implantate weisen deutlich geringere oder sogar gar keine Abstoßungsreaktionen auf. Aufgrund der optimierten Paßform des Implantats können die körpereigenen Knorpelzellen besonders gut einwachsen. Das Implantatmaterial kann homogen sein oder aus unterschiedlichen Schichten bestehen, die jedoch nicht alle zur Beimpfung mit Knorpelzellen geeignet sein müssen. Beispielsweise kann auch eine Deck- oder Schutzschicht für den mit Knorpelzellen beimpften Träger vorgesehen sein.

Damit das Knorpelersatzimplantat Knorpelzellen besonders gut aufnehmen kann, ist es vorteilhaft, wenn als Trägermaterial ein Vlies verwendet wird. Ein Vlies ist leicht, weist ausreichend Hohlräume auf und ist zudem besonders gut bearbeitbar.

Grundsätzlich wäre es denkbar, den präparierten Träger direkt in den Knorpeldefekt einzusetzen, insbesondere ohne ihn vor dem Implantieren bzw. Einsetzen mit Knorpelzellen zu beimpfen. Bei dieser Operationstechnik wird vorzugsweise die subchondrale Platte des Knorpeldefekts wie eingangs beschrieben mikrofrakturiert, so daß es zum Einbluten autologer Zellen, insbesondere aus dem Fettmarkgewebe des darunterliegenden spongiösen Knochens, in das Trägermaterial kommt, die bei der Knorpelregeneration eine entscheidende Rolle spielen. Allerdings ist es vorteilhaft, wenn der Träger nach dem Präparieren und vor dem Implantieren mit Knorpelzellen beimpft wird. Es ist so möglich, gezielt Knorpelzellen in den Träger einzubringen, um so direkt nach der Implantation ein Zellwachstum möglichst sicher und schnell gewährleisten zu können.

Das Einwachsen des Knorpelersatzimplantats wird zusätzlich verbessert und mögliche Abstoßungsreaktionen des Körpers werden verringert oder sogar ganz ausgeschaltet, wenn vor der Implantation des Knorpelersatzimplantats Knorpelzellen aus dem menschlichen oder tierischen Körper entnommen werden, wenn die entnommenen körpereigenen Knorpelzellen im Labor vermehrt werden und wenn die vermehrten körpereigenen Knorpelzellen zum Beimpfen des Trägers bereitgestellt werden.

Damit nur die unbedingt erforderliche Menge an Knorpelzellen bereitgestellt werden muß, was insbesondere den Herstellungsaufwand und die damit verbundenen Kosten minimiert, ist es günstig, wenn aus dem Defektdatensatz das Defektvolumen des Knorpeldefekts bestimmt wird und wenn ein Implantationsvolumen von Knorpelzellen bereitgestellt wird, wobei das Implantationsvolumen ausreichend ist, um den Träger für eine Implantation des Knorpelersatzimplantats ausreichend mit Knorpelzellen zu beimpfen. Es werden also nur so viel Knorpelzellen bereitgestellt, wie tatsächlich zur Beimpfung des Knorpelersatzimplantats erforderlich sind. Dies hat zudem den Vorteil, daß bei kleinen Defekten eine Implantation des Knorpelersatzimplantats schon nach einer viel kürzeren Zeit gerechnet ab Entnahme der körpereigenen Knorpelzellen erfolgen kann.

Das bereitzustellende Implantationsvolumen von Knorpelzellen kann weiter verringert werden, wenn das bereitgestellte Trägermaterial Hohlräume zur Aufnahme von Knorpelzellen aufweist, wenn die Hohlräume des Trägers ein Hohlraumvolumen aufweisen und wenn ein Implantationsvolumen bereitgestellt wird, das dem Hohlraumvolumen oder in etwa dem Hohlraumvolumen des Trägers entspricht. Bei bekanntem Trägermaterial läßt sich auf einfache Weise das Hohlraumvolumen des Implantats bestimmen. Als Implantationsvolumen reicht dann ein Volumen aus, das dem Hohlraumvolumen oder in etwa dem Hohlraumvolumen des Trägers entspricht.

Damit das Implantat auf einfache Weise präpariert werden kann, ist es günstig, wenn der Defektdatensatz mit einer Datenverarbeitungseinrichtung zu einem Präparierdatensatz verarbeitet wird, wobei der Präparierdatensatz Daten zur Beschreibung insbesondere von Form, Fläche und/oder Volumen des zu präparierenden Implantats enthält. Mit der Datenverarbeitungseinrichtung läßt sich der Präparierdatensatz besonders schnell erzeugen, so daß ein Knorpelersatzimplantat direkt nach Bestimmen des Defektdatensatzes präpariert werden kann, beispielsweise noch während eines chirurgischen Eingriffs.

Günstigerweise wird zum Bestimmen des Defektdatensatzes ein minimalinvasiver Zugang in den menschlichen oder tierischen Körper eröffnet. Durch den minimalinvasiven Zugang läßt sich ein Operationstrauma minimieren. Außerdem wird das Infektionsrisiko deutlich verringert.

Vorteilhaft ist es, wenn vor dem Bestimmen des Defektdatensatzes ein Rand des Defekts präpariert wird. Durch die Präparation des Randes des Defekts, insbesondere durch das Entfernen von losen, nicht mit der subchondralen Knochenplatte verbundenen Fragmenten oder Beschneiden des Defektrandes, kann eine Spaltbildung zwischen dem Implantat und dem Defektrand verhindert werden. So wird genau der Rand des Defekts bestimmt, an den das Implantat nach dem Einsetzen angrenzt, sodaß dieses den Defekt optimal ausfüllend präpariert werden kann.

Gemäß einer bevorzugten Variante des erfindungsgemäßen Verfahrens kann vorgesehen sein, daß ein Meßinstrument bereitgestellt, an welchem ein von einer Nachweisvorrichtung eines Navigationssystems detektierbares Referenzelement anordenbar ist zum Bestimmen von Lage und Orientierung des Meßinstruments im Raum, und daß mit dem Meßinstrument der Defekt zum Bestimmen des Defektdatensatzes vermessen und gleichzeitig die Lage und die Orientierung des Meßinstruments im Raum bestimmt wird. Durch Verwendung eines solchen Meßinstruments kann die Größe des Defekts besonders genau bestimmt werden, es können zum Beispiel sogar Raumkoordinaten des Defekts im jeweiligen Bezugssystem angegeben werden, insbesondere auch in Echtzeit. Dadurch kann vermieden werden, daß beispielsweise zu wenig Knorpelzellen zum Beimpfen eines Knorpelersatzimplantats bereitgestellt werden. Dies könnte man zwar auch vermeiden, indem ein deutlich größeres als tatsächlich benötigtes Implantationsvolumen an Knorpelzellen bereitgestellt wird, doch werden dadurch auch die Kosten für einen Eingriff deutlich höher. Außerdem nimmt die Qualität der Zellen mit Zunahme der Passagierung ab, das heißt, je geringer die Zahl der erforderlichen Passagen umso besser die Qualität der Zellen. Durch das genaue Vermessen läßt sich ferner auch die Ausbildung eines Spalts zwischen dem Implantat und einer Begrenzung des Defekts nach Implantation des Implantats vermeiden.

Besonders vorteilhaft ist es, wenn das Meßinstrument durch den minimalinvasiven Zugang in den menschlichen oder tierischen Körper eingeführt wird. Durch einen minimalinvasiven Zugang kann dann nicht nur der Defekt vermessen, sondern auch behandelt werden, das heißt, daß insbesondere auch das Implantat durch den Zugang eingesetzt werden kann.

Günstig ist es, wenn das Meßinstrument eine Tastspitze aufweist und wenn zum Ausmessen des Defekts ein Rand oder eine Begrenzung des Defekts mit der Tastspitze palpiert wird. Diese Vorgehensweise ermöglicht es einem erfahrenen Operateur oder Arthroskopeur sogar, den Defekt zu vermessen, ohne ihn direkt zu sehen oder auch wenn eine eingeführte Optik ein verzerrtes Bild des Defekts darstellt. Er muß dabei nur mit der Tastspitze an einem Rand oder einer Begrenzung des Defekts entlangfahren. Die Begrenzungslinie wird dann durch die Nachweisvorrichtung aufgezeichnet, so daß der bestimmte Defektdatensatz alle erforderlichen Daten enthält, um ein dem Defekt entsprechendes Implantat zu präparieren.

Günstig ist es, wenn der Defekt berührungslos ausgemessen wird. Dies hat den Vorteil, daß der Defekt durch das Ausmessen nicht weiter vergrößert wird. Beispielsweise kann elektromagnetische Strahlung, insbesondere sichtbares Licht, oder Ultraschall zum Ausmessen des Defekts genutzt werden.

Damit ein Operateur eine noch bessere Vorstellung vom Defekt erhalten kann, ist es vorteilhaft, wenn das Meßinstrument eine optische Bilderfassungseinheit aufweist und wenn mit der Bilderfassungseinheit mindestens ein Defektbild des Defekts aufgenommen wird. Ein Operateur kann somit den Defekt auch direkt in Echtzeit sehen und mit einer entsprechenden optischen Ausstattung beurteilen, zudem läßt sich der Defekt so berührungslos vermessen, zum Beispiel durch Auswerten des Defektbilds.

Günstig ist es, wenn von der Bilderfassungseinheit ein Bilddatensatz des mindestens einen Defektbilds bereitgestellt wird und wenn der Bilddatensatz zum Defektdatensatz verarbeitet wird. Die von der Bilderfassungseinheit bereitgestellten Daten lassen sich auf einfache Weise zum Defektdatensatz verarbeiten, sodaß alle geometrischen Informationen des Defekts bekannt sind.

Besonders schnell läßt sich der Defektdatensatz erhalten, wenn der Bilddatensatz mit einer Datenverarbeitungseinrichtung zum Defektdatensatz verarbeitet wird.

Grundsätzlich könnte als Bilderfassungseinheit eine, beispielsweise für sichtbares Licht optimierte, Kamera mit oder ohne zusätzliche optische Bauelemente verwendet werden. Alternativ wären günstigerweise auch Mikrowellenstrahlung nachweisende oder erfassende Bilderfassungseinheiten, Bilderfassungseinheiten für die optische Kohärenz Tomographie (OCT) sowie Ultraschall-Bilderfassungseinheiten verwendbar. Vorteilhafterweise werden als Bilderfassungseinheit Infrarotsensoren verwendet. Diese haben den Vorteil, daß zur Bilderfassung nicht zwingend eine Beleuchtungsquelle erforderlich ist. Dadurch reicht auch ein besonders kleiner minimalinvasiver Zugang aus, um den Defektdatensatz zu bestimmen.

Das Implantat läßt sich in vorteilhafter Weise präparieren, wenn ein Bearbeitungswerkzeug zum Bearbeiten des Implantatmaterials bereitgestellt wird und wenn das Implantatmaterial mit dem Bearbeitungswerkzeug unter Verwendung des Defektdatensatzes bearbeitet wird. Bei der Bearbeitung des Implantatmaterials wird der Defektdatensatz herangezogen, das heißt das Implantat wird nicht unabhängig von dem bereitgestellten Defektdatensatz, sondern gezielt unter Berücksichtigung desselben präpariert.

Dabei kann es vorteilhaft sein, wenn das Bearbeitungswerkzeug ein von der Nachweisvorrichtung detektierbares Referenzelement aufweist und wenn das Implantatmaterial mit dem Bearbeitungswerkzeug unter gleichzeitiger Detektion von Lage und Orientierung des Bearbeitungswerkzeugs bearbeitet wird. Eine Bearbeitung des Implantatmaterials kann auf diese Weise mit dem Navigationssystem nicht nur direkt oder indirekt überwacht und kontrolliert, sondern auch geführt werden. Beispielsweise wäre es denkbar, eine vom Bearbeitungswerkzeug abzufahrende Sollkurve zusammen mit der gleichzeitig bestimmten Ist-Bewegungsbahn des Bearbeitungswerkzeugs an einer Anzeigevorrichtung anzuzeigen. Eine Person, die das Implantat präpariert, kann dann sofort erkennen, wie das Bearbeitungswerkzeug zu führen ist, um das Implantat in gewünschter Weise zu präparieren.

Günstigerweise wird als Bearbeitungswerkzeug ein Markierstift zum Übertragen des Defektdatensatz auf das Implantatmaterial bereitgestellt. Beispielsweise kann mit einem Markierstift ein flächiges Implantatmaterial mit der Kontur des Defektes versehen werden, sodaß nach dem Markieren oder anzeichnen des Implantatmaterials das Implantat in gewünschter Weise aus dem Implantatmaterial herausgetrennt werden kann.

Gemäß einer bevorzugten Variante des erfindungsgemäßen Verfahrens kann vorgesehen sein, daß als Bearbeitungswerkzeug eine Schneidvorrichtung zum Ausschneiden des Trägers aus dem Implantatmaterial unter Verwendung des Defektdatensatzes bereitgestellt wird. In Kombination mit einem Markierstift, oder auch ohne einen Markierstift, kann mit der Schneidvorrichtung das Implantatmaterial direkt so präpariert werden, daß ein Implantat gewünschter Form und Größe entsteht.

Grundsätzlich wäre es denkbar, das Bearbeitungswerkzeug von einer Bedienperson manuell bedienen zu lassen und damit das Implantat zu präparieren. Um das Implantat vollautomatisch präparieren zu lassen, ist es günstig, wenn eine Bearbeitungsvorrichtung zum Bearbeiten des Implantatmaterials bereitgestellt wird, wenn der Defektdatensatz auf die Bearbeitungsvorrichtung übertragen wird und wenn das Implantatmaterial mit der Bearbeitungsvorrichtung bearbeitet wird. Beispielsweise kann die Bearbeitungsvorrichtung eine Fräs- eine Schneidemaschine oder eine Stanze sein, die eine Datenverarbeitungseinrichtung umfaßt und daß der Defektdatensatz zur Ansteuerung der Bearbeitungsvorrichtung verwendet werden kann, um ein Implantat gewünschter Form und Größe zu präparieren.

Vorteilhaft ist es, wenn die bereitgestellte Bearbeitungsvorrichtung das Bearbeitungswerkzeug umfaßt. Es ist dann nicht zwingend erforderlich, das Bearbeitungswerkzeug zu navigieren. Allerdings kann ein navigiertes Bearbeitungswerkzeug vorteilhaft sein, um Fehler bei der Präparierung des Implantats zu vermeiden.

Die nachfolgende Beschreibung bevorzugter Ausführungsformen der Erfindung dient im Zusammenhang mit der Zeichnung der näheren Erläuterung. Es zeigen:
- Figur 1:: eine erste Variante eines chirurgischen Systems mit einem Meßinstrument mit Tastspitze;

- Figur 2:: ein zweites Ausführungsbeispiel eines chirurgischen Systems mit einem Meßinstrument mit Bilderzeugungseinheit;
- Figur 3:: ein chirurgisches System mit einem Bearbeitungswerkzeug zum Markieren eines Implantatmaterials;
- Figur 4:: ein chirurgisches System mit einem navigierten Bearbeitungs-werkzeug zum Heraustrennen eines Trägers oder eines Eratzimplantats aus einem Trägermaterial; und
- Figur 5:: eine schematische Darstellung des erfindungsgemäßen Verfahrens.

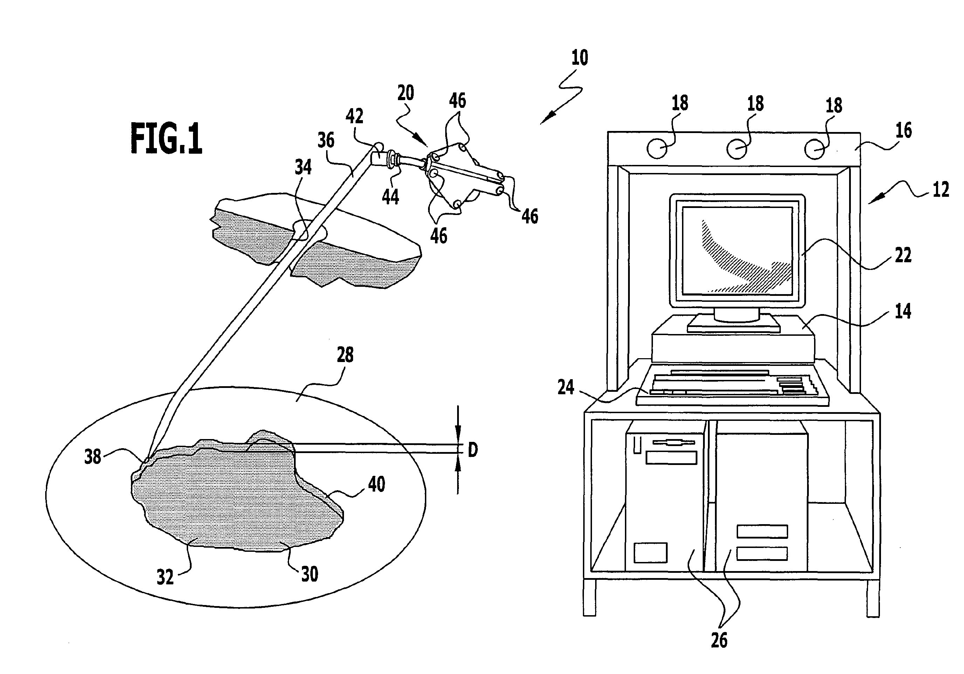

In Figur 1 ist ein insgesamt mit dem Bezugszeichen 10 versehenes chirurgisches System dargestellt, welches ein insgesamt mit dem Bezugszeichen 12 versehenes Navigationssystem umfaßt.

Das Navigationssystem 12 weist eine von einem als Datenverarbeitungseinrichtung dienenden Rechner 14 gesteuerte Sende- und Empfangsstation 16 auf, welche mehrere Sende- und Empfangseinheiten 18 umfaßt zum Aussenden und Empfangen von elektromagnetischer Strahlung, sowohl im sichtbaren wie auch im Infrarotbereich, oder Ultraschall, die von einem Referenzelement 20 ausgesandt oder reflektiert wird. Das chirurgische System 10 und/oder das Navigationssystem 12 umfassen ferner eine Anzeigevorrichtung in Form eines Bildschirms 22 sowie ein Eingabegerät in Form einer Tastatur 24. Zur Erhöhung der Leistungsfähigkeit des beziehungsweise der Systeme 10 und 12 können weitere Recheneinheiten 26 mit dem Rechner 14 zusammenwirken. Um die Funktion des Navigationssystems 12 in optimaler Weise zu gewährleisten, sind mindestens drei voneinander räumlich beabstandete Sende- und Empfangseinheiten 18 vorgesehen.

Zum Behandeln, insbesondere zum Ausfüllen eines aufgrund von Verschleiß und/oder eines chirurgischen Angriffs entstandenen Defekts in einem Knochen und/oder einem Knorpel eines Gelenks eines menschlichen oder tierischen Körpers wird der Defekt mit Hilfe des chirurgischen Systems ausgemessen. Als ein möglicher Behandlungsfall ist in Figur 1 ein Ausschnitt eines Gelenkknorpel 28 eines nicht näher dargestellten menschlichen oder tierischen Gelenks mit dem Bezugszeichen 28 versehen. Infolge von Verschleiß, eines Unfalls oder eines chirurgischen Eingriffs ist der Gelenkknorpel 28 beschädigt, er weist also einen Knorpeldefekt 30 auf, welcher in der Regel eine einer Dicke der Knorpelschicht entsprechende Dicke D aufweist. Die Dicke D kann konstant oder im wesentlichen konstant über den gesamten Knorpeldefekt 30 sein. Allerdings kann sie auch abhängig von einer natürlichen Dicke des Gelenkknorpels 28 etwas unterschiedlich sein. In besonders kritischen Fällen ist der Gelenkknorpel 28 bis auf die darunterliegende subchondrale Platte 32 beschädigt.

Zur Bestimmung insbesondere von Größe, Form, Fläche und/oder Volumen des Knorpeldefekts 30 wird dieser ausgemessen. Zu diesem Zweck wird zunächst ein in Figur 1 schematisch angedeuteter minimalinvasiver Zugang 34 in den menschlichen oder tierischen Körper eröffnet. Mit einem als Meßinstrument dienenden Tasthaken 36 wird der Knorpeldefekt 30 vermessen. Ein distales Ende des Tasthakens 36 ist in Form einer Tastspitze 38 ausgebildet, mit der ein Rand 40 oder eine anderweitige Begrenzung des Knorpeldefekts 30 abgetastet oder palpiert werden kann. An einem proximalen Ende des Tasthakens 36 ist ein Kupplungszapfen 42 angeordnet, über welchen der Tasthaken 36 mit einem Referenzelement 20 verbindbar ist, welches ein zum Kupplungszapfen 42 korrespondierendes Kupplungselement 44 aufweist. Das Referenzelement 20 weist mindestens drei passive oder aktive Positionselemente 46 auf. Passive Positionselemente 46 sind geeignet, elektromagnetische Strahlung zu reflektieren oder auch verzögert abzustrahlen, die von den Sende- und Empfangseinheiten 18 ausgesandt wird. Aktive Positionselement 46 dagegen können selbst elektromagnetische Strahlung erzeugen und aussenden, die von den Sende- und Empfangseinheiten 18 empfangen werden kann. Werden nur aktive Positionselemente 46 verwendet, können anstelle der Sende- und Empfangseinheiten 18 auch reine Empfangseinheiten vorgesehen werden.

Die räumliche Lage der Positionselemente 46 kann in Echtzeit vom Navigationssystem 12 erfaßt werden. Da zudem die Lage der Positionselemente 46 relativ zum Tasthaken 36, insbesondere zur Tastspitze 38 bekannt sind, kann aus der ermittelten Lage der Positionselemente 46 die räumliche Lage und die Orientierung des Tasthakens 36 im Raum bestimmt werden. Wird also mit der Tastspitze 38 des Tasthakens 36 der Rand 40 des Knorpeldefekts 30 palpiert, so kann mit dem Navigationssystem 12 ein Defektdatensatz aufgenommen werden, der Daten zur Angabe der räumlichen Lage des Rands 40 im Raum enthält. Aus diesem Defektdatensatz lassen sich dann beispielsweise mit dem Rechner 14 und/oder den Recheneinheiten 26 Defektdaten zur Angabe insbesondere von Form, Größe, Fläche und/oder Volumen des Knorpeldefekts 30 ermitteln.

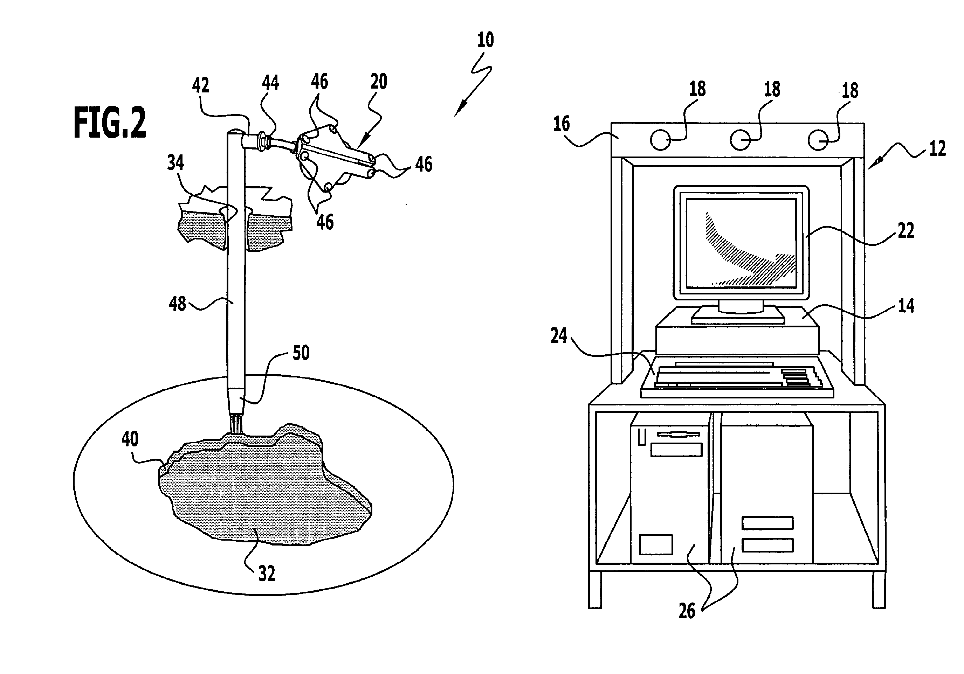

Alternativ zum Tasthaken 36 kann auch ein Meßinstrument 48 verwendet werden, wie es in Figur 2 schematisch dargestellt ist. Es weist eine an seinem distalen Ende angeordnete Bilderfassungseinheit 50 auf, die beispielsweise eine oder mehrere Infrarotsensoren umfaßt. Die Bilderfassungseinheit könnte auch durch einen Lichtwellenleiter mit einem Eintrittsfenster am distalen Ende des Instruments und eine mit dem proximalen Ende des Instruments verbindbare oder direkt am distalen Ende des Instruments angeordnete Kamera gebildet werden. Das Meßinstrument 48 ist ebenfalls über einen Kupplungszapfen 42 mit dem Kupplungselements 44 des Referenzelements 20 verbindbar. Auch das Meßinstrument 48 kann über einen minimalinvasiven Zugang 34 in den menschlichen oder tierischen Körper eingeführt werden. Mittels der Bilderfassungseinheit 50 kann der Knorpeldefekt 30 optisch, das heißt berührungslos, ausgemessen werden. Hierzu wird die Bilderfassungseinheit 50 über den Knorpeldefekt 30 bewegt und quasi abgescannt. Da das Meßinstrument 48, ebenso wie der Tasthaken 36, beim Ausmessen des Knorpeldefekts 30 navigiert wird, das heißt seine Lage und Orientierung wird vom Navigationssystem 12 erfaßt, kann jedem einzelnen von der Bilderfassungseinheit 50 aufgenommenen Defektbild oder Zeilenscans des Knorpeldefekts 30 dessen räumliche Lage und Orientierung zugeordnet werden. Aus einer Vielzahl von mit der Bilderfassungseinheit 50 aufgenommener Einzelaufnahmen oder Zeilenscans des Knorpeldefekts 30 läßt sich so mit dem Rechner 14 und/oder den Recheneinheiten 26 ein Bilddatensatz ermitteln, welcher mit dem Rechner 14 und/oder den Recheneinheiten 26 zu einem Defektdatensatz verarbeitbar ist. Der Defektdatensatz kann insbesondere auch angeben, wie tief der Defekt ist, also ob der Defekt nur den Knorpel oder auch die darunterliegende subchondrale Platte oder sogar den Knochen betrifft. Ist letzteres der Fall, dann kann vor dem Einsetzen des Implantats der Defekt an der subchondralen Platte und/oder am Knochen durch Knochenspäne oder Knochenersatzmaterial aufgefüttert werden, um den Erfolg der Behandlung des Knorpeldefekts nicht zu gefährden. Idealerweise sind die mit dem Tasthaken 36 und mit dem Meßinstrument 48 gewonnenen Defektdatensätze identisch.

Das chirurgische System 10 umfaßt ferner ein Bearbeitungswerkzeug in Form eines Markierstifts 52, welcher dazu verwendet werden kann, auf einem Implantatmaterial 54, beispielsweise einem Collagen-Vlies, die Kontur 56 des zu präparierenden Implantats 58 zu markieren. Zu diesem Zweck weist der Markierstift 52 eine Spitze 60 auf, mit der das Implantatmaterial 54 beispielsweise beschriftet, also mit einer Linie oder dergleichen versehen oder, je nach Art des Implantatmaterials, anderweitig markiert werden kann, zum Beispiel durch Versehen mit einer Kratzspur. Das Implantatmaterial 54 weist vorzugsweise eine Dicke D_{I} auf, die der Dicke D des Knorpeldefekts 30 entspricht. Das gekennzeichnete Implantatmaterial 54 kann nach dem Markieren beispielsweise mit einem Schneidwerkzeug zum Heraustrennen des Implantats 58 weiterbearbeitet werden. Dieses entspricht in Form, Größe, Kontur und Volumen dem Knorpeldefekt 30.

Zum Markieren des Implantatmaterials 54 ist der Markierstift 52 mit einem Referenzelement 20 verbindbar, vorzugsweise über einen Kupplungszapfen 52, welcher am proximalen Ende des Markierstifts 52 angeordnet ist, und ein korrespondierendes Kupplungselement des Referenzelements 20. Dadurch ist eine Bewegung der Spitze 60 des Markierstifts 52 mit dem Navigationssystem 12 navigierbar. Insbesondere ist es möglich, eine Sollkontur 62 auf dem Bildschirm 22 darzustellen, die mit dem Markierstift 52 nachgefahren werden sollte. Zur Kontrolle kann gleichzeitig eine Istkontur beziehungsweise Ist-Bewegungsbahn 64 der Spitze 60 auf dem Bildschirm zusammen mit der Sollkontur 62 ausgegeben werden. Eine Person, die das Implantatmaterial 54 für eine weitere Bearbeitung markieren soll, erkennt dann sofort, wie der Markierstift 52 weiter zu bewegen ist.

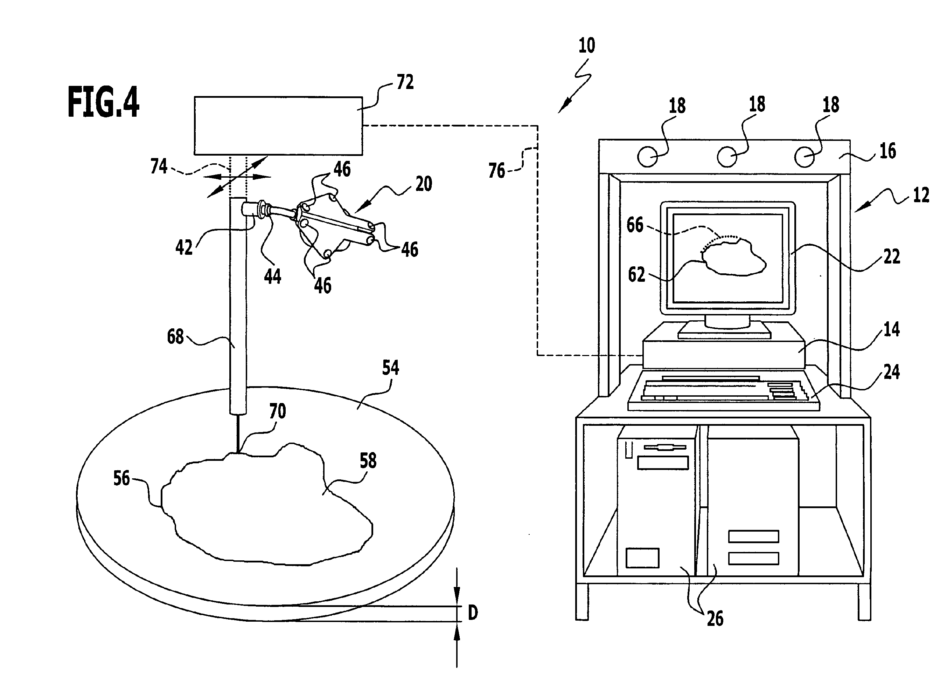

Im Zusammenhang mit Figur 4 wird nachfolgend näher beschrieben, wie das Implantat 58 alternativ aus einem Implantatmaterial 54 präpariert werden kann.

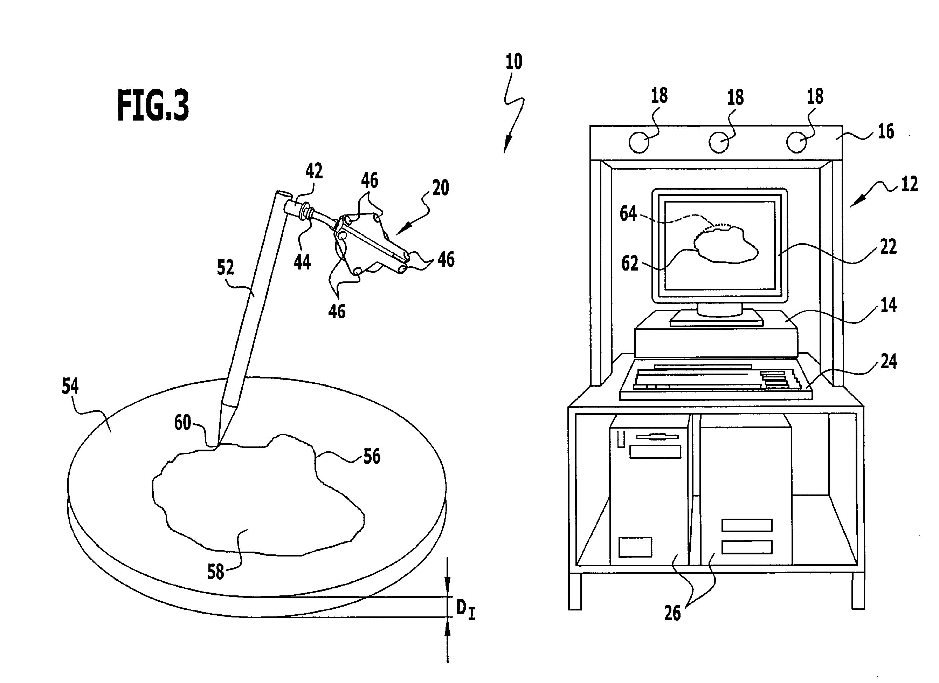

Hierzu kann anstelle des Markierstifts 52 ein Schneidwerkzeug 68 eingesetzt werden, welches an seinem distalen Ende mit einer Klinge oder Schneidspitze 70 versehen ist, welche geeignet ist, das Implantatmaterial zu bearbeiten. Alternativ könnte das Schneidwerkzeug auch ein elektrisch betätigbares, ein Strom oder Licht, beispielsweise ein Laser, zum Schneiden verwendendes Werkzeug sein. Das Schneidwerkzeug 68 ist ebenfalls mit einem Kupplungszapfen 42 versehen, welcher korrespondierend zum Kupplungselement 42 des Referenzelements 20 ausgebildet ist. Damit kann, wie bereits im Zusammenhang mit Figur 3 eine Bewegung des Schneidwerkzeugs 68, falls gewünscht, navigiert werden. Wird am Bildschirm 22 die Sollkontur 62 des Implantats 58 angezeigt, so kann einer Person, die das Implantatmaterial 54 bearbeitet, gleichzeitig eine Istschneidbahn 66 der Schneidspitze 70 zusammen mit der Sollkontur 62 angezeigt werden. Die präparierende Person kann dann die Ist-Schneidbahn 66 derart vorgeben, daß sie möglichst identisch mit der Sollkontur 62 ist.

Das Implantatmaterial 54 kann jedoch nicht nur manuell bearbeitet werden, wie oben beschrieben, sondern auch maschinell, und zwar mit einer in Figur 4 schematisch dargestellten Bearbeitungsvorrichtung 72. Die Bearbeitungsvorrichtung 72 läßt sich beispielsweise mit dem Schneidwerkzeug 68 über eine in Figur 4 gestrichelt eingezeichnete Verbindungseinheit 74 koppeln, so daß das Schneidwerkzeug 68 in beliebiger Richtung, angedeutet durch die Richtungspfeile in Figur 4, über das Implantatmaterial 54 hinwegbewegt werden kann. Die Bearbeitungseinrichtung 72 kann beispielsweise über den Rechner 14 angesteuert werden. Zu diesem Zweck ist der Rechner 14 mit der Bearbeitungseinrichtung 72 über eine Steuerleitung 76 verbunden, die in Figur 4 gestrichelt eingezeichnet ist. Der Vorteil elektrisch betätigbarer, Strom oder Licht, beispielsweise in Form eines Lasers, zum Schneiden verwendende Schneidwerkzeuge ist darin zu sehen, daß eine Energiezufuhr des Schneidwerkzeugs sofort unterbrochen und dadurch der Schneidvorgang gestoppt werden kann, falls die Ist-Schneidbahn 66 von der Sollkontur 62 abweicht.

Bei Verwendung einer Bearbeitungseinrichtung 72 ist es nicht unbedingt erforderlich, das Schneidwerkzeug 78 zu navigieren. Vielmehr genügt es, den Defektdatensatz zu verwenden. Hierzu kann insbesondere vorgesehen sein, den Defektdatensatz in einen Bearbeitungsdatensatz zu verarbeiten. Entweder der Bearbeitungsdatensatz oder der Defektdatensatz können zur Ansteuerung der Bearbeitungsvorrichtung 72 verwendet werden.

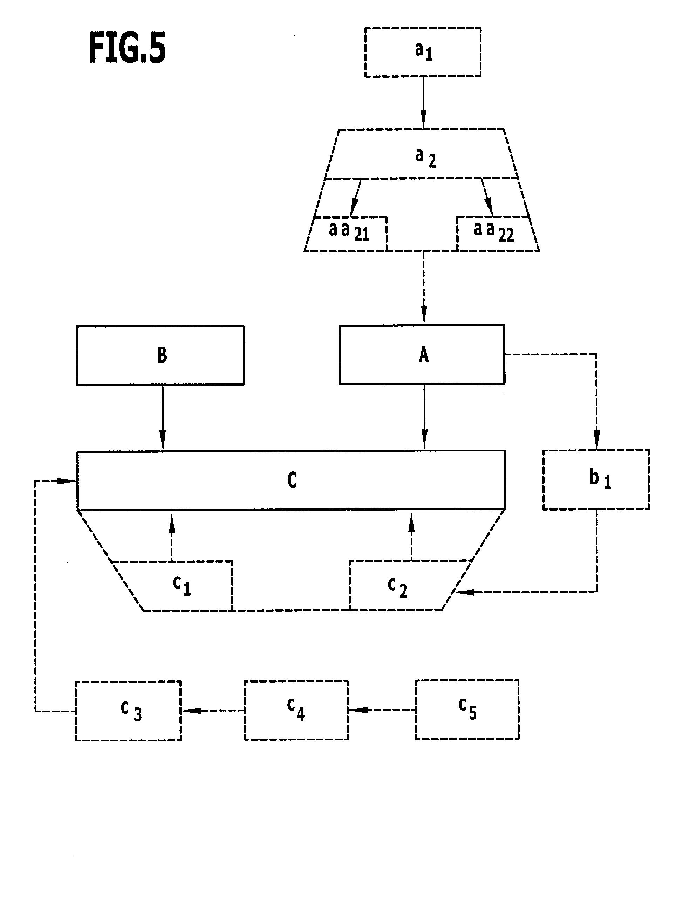

Schematisch ist das erfindungsgemäße Verfahren zum Präparieren eines Implantats in Figur 5 dargestellt. Die mit großen und kleinen Buchstaben bezeichneten Verfahrensschritte sind nachfolgend beschrieben. Wesentliche Verfahrensschritte sind:
- das Bereitstellen eines Datensatzes, welcher Daten zur Beschreibung von Form, Fläche und/oder Volumen des Defekts enthält (A),
- das Bereitstellen mindestens eines Implantatmaterials (B) und .
- das Präparieren des dem Defekt in Form und Größe entsprechenden Implantats aus dem Implantatmaterial unter Verwendung des Defektdatensatzes (C).

Die Verfahrensschritte (A) bis (C) sind für das erfindungsgemäße Verfahren essentiell.

Zur Bereitstellung des Defektdatensatzes kann insbesondere wie folgt vorgegangen werden:
- Eröffnen eines mininmalinvasiven Zugangs zum menschlichen oder tierischen Körper (a₁),
- Präparation eines Rand des Defekts, insbesondere Entfernen von losen, nicht mit der subchondralen Knochenplatte verbundenen Fragmenten oder Beschneiden eines Rand des Defekts,
- Bestimmen des Defektdatensatzes mit einem navigierten Meßinstrument (a₂),
- wobei der Defektdatensatz berührungslos mit einer Bilderfassungseinheit (aa₂₁) oder tastend mit einem Tastinstrument (aa₂₂) bestimmt werden kann.

Das Präparieren des Implantats kann mittels eines navigierten Bearbeitungswerkzeugs (c₁) oder mit einer Bearbeitungseinrichtung (c₂) geschehen, wobei die Bearbeitungseinrichtung unter Verwendung des Defektdatensatzes gesteuert werden kann. Zu diesem Zweck kann der Defektdatensatz mit einer Datenverarbeitungseinrichtung zu einem Präparierdatensatz verarbeitet werden (b₁), der geeignet ist zur Ansteuerung der Bearbeitungseinrichtung verwendet zu werden.

Zusätzlich zur reinen formgebenden Präparierung des Implantats können vorab durch einen minimalinvasiven Zugang dem Patienten körpereigene Knorpelzellen entnommen werden (c₃). Die entnommenen Körperzellen können im Labor vermehrt werden, bis ein erforderliches Implantationsvolumen der körpereigenen Knorpelzellen zur Verfügung steht (c₄). Anschließend können die vermehrten körpereigenen Knorpelzellen zum Beimpfen des Knorpelersatzimplantats eingesetzt werden (c₅).

Der Einsatz des erfindungsgemäßen Verfahrens und auch des erfindungsgemäßen chirurgischen Systems ist nicht auf die Behandlung von Knorpeldefekten beschränkt. Auch Defekte an Knochen oder anderen, im wesentlichen aus härterem Gewebe bestehenden Teilen des menschlichen Körpers können auf die beschriebene Art und Weise mit einem Implantat ausgefüllt werden.

Aus dem ermittelten Volumen des Knorpeldefekts 30 läßt sich bei Kenntnis des Implantatmaterials 54, das vorzugsweise Hohlräume zur Aufnahme von körpereigenen, im Labor vermehrten Knorpelzellen aufweist, ein von den Knorpelzellen zu füllendes Hohlraumvolumen berechnen. Bei Kenntnis des Hohlraumvolumens kann die Vermehrung der Knorpelzellen im Labor optimiert vorgenommen werden, das heißt es müssen nur so viele Knorpelzellen gezüchtet werden, damit das Implantat 58 ausreichend mit Knorpelzellen beimpft werden kann.

## Patentansprüche

1. Chirurgisches System (10) zum Präparieren eines Implantats (58) zum Ausfüllen eines aufgrund eines Traumas und/oder Degeneration entstandenen Defekts (30) an einem Knochen und/oder einem Knorpel eines menschlichen oder tierischen Körpers, wobei mit dem System (10) ein bereitgestellter Defektdatensatz verarbeitbar ist, welcher Daten insbesondere zur Beschreibung von Form, Fläche, Höhe und/oder Volumen des Defekts (30) enthält, und wobei mit dem System (10) aus mindestens einem bereitgestellten Implantatmaterial (54) das dem Defekt (30) in Form und Größe entsprechende Implantat (58) unter Verwendung des Defektdatensatzes präparierbar ist.

2. System nach Anspruch 1, **dadurch gekennzeichnet, daß** das Implantat (58) ein Knorpelersatzimplantat für die autologe Chondrocyten Transplantation (ACT) ist und daß mit dem Knorpelersatzimplantat der einen Knorpeldefekt bildende Defekt (30) ausfüllbar ist.

3. System nach Anspruch 2, **dadurch gekennzeichnet, daß** das Implantatmaterial (54) ein für die Beimpfung mit Knorpelzellen geeignetes Trägermaterial ist, daß das Knorpelersatzimplantat (58) einen Träger für die Knorpelzellen umfaßt und daß der Träger aus dem Trägermaterial (54) präpariert ist.

4. System nach Anspruch 3, **dadurch gekennzeichnet, daß** das Trägermaterial (58) ein Vlies ist.

5. System nach einem der Ansprüche 3 oder 4, **dadurch gekennzeichnet, daß** der Träger (58) mit Knorpelzellen beimpfbar ist.

6. System nach Anspruch 5, **dadurch gekennzeichnet, daß** der Träger mit Knorpelzellen beimpfbar ist, die im Labor vermehrte körpereigene Knorpelzellen sind, welche vor dem Einsetzen des Knorpelersatzimplantats (58) aus dem menschlichen oder tierischen Körper entnommen wurden.

7. System nach einem der Ansprüche 3 bis 6, **dadurch gekennzeichnet, daß** aus dem Defektdatensatz das Defektvolumen des Knorpeldefekts (30) bestimmbar ist und daß ein bereitgestelltes Implantationsvolumen von Knorpelzellen ausreicht, um den Träger für eine Implantation des Knorpelersatzimplantats (58) ausreichend mit Knorpelzellen zu beimpfen.

8. System nach Anspruch 7, **dadurch gekennzeichnet, daß** das bereitgestellte Trägermaterial Hohlräume zur Aufnahme von Knorpelzellen aufweist, daß die Hohlräume des Trägers (58) ein Hohlraumvolumen aufweisen und daß ein bereitgestelltes Implantationsvolumen dem Hohlraumvolumen oder in etwa dem Hohlraumvolumen des Trägers (58) entspricht.

9. System nach einem der voranstehenden Ansprüche, **dadurch gekennzeichnet, daß** eine Datenverarbeitungseinrichtung (14, 26) vorgesehen ist zum Verarbeiten des Defektdatensatzes mit der Datenverarbeitungseinrichtung (14, 26) zu einem Präparierdatensatz, wobei der Präparierdatensatz Daten insbesondere zur Beschreibung von Form, Fläche, Höhe und/oder Volumen des zu präparierenden Implantats (58) enthält.

10. System nach einem der voranstehenden Ansprüche, **dadurch gekennzeichnet, daß** ein chirurgisches Instrument vorgesehen ist zum Eröffnen eines minimalinvasiven Zugangs (34) in den menschlichen oder tierischen Körper zum Bestimmen des Defektdatensatzes.

11. System nach einem der voranstehenden Ansprüche, **dadurch gekennzeichnet, daß** ein Navigationssystem (12) mit einer Nachweisvorrichtung (16, 18) vorgesehen ist, daß ein Meßinstrument (36, 48) vorgesehen ist, an welchem ein von der Nachweisvorrichtung (16, 18) des Navigationssystems (12) detektierbares Referenzelement (20) anordenbar ist zum Bestimmen von Lage und Orientierung des Meßinstruments (36, 48) im Raum und daß mit dem Meßinstrument (36, 48) der Defekt (30) zum Bestimmen des Defektdatensatzes ausmeßbar und gleichzeitig mit dem Navigationssystem (12) die Lage und die Orientierung des Meßinstruments (36, 48) im Raum bestimmbar sind.

12. System nach Anspruch 11, **dadurch gekennzeichnet, daß** das Meßinstrument (36, 48) durch einen minimalinvasiven Zugang (34) in den menschlichen oder tierischen Körper einführbar ist.

13. System nach einem der Ansprüche 11 oder 12, **dadurch gekennzeichnet, daß** das Meßinstrument (36) eine Tastspitze (38) aufweist und daß zum Ausmessen des Defekts (30) ein Rand (40) oder eine Begrenzung des Defekts (30) mit der Tastspitze (38) palpierbar ist.

14. System nach einem der Ansprüche 11 oder 12, **dadurch gekennzeichnet, daß** das Meßinstrument (48) derart ausgebildet ist, daß der Defekt (30) berührungslos ausmeßbar ist.

15. System nach einem der Ansprüche 11 bis 14, **dadurch gekennzeichnet, daß** das Meßinstrument (48) eine optische Bilderfassungseinheit (50) aufweist und daß mit der Bilderfassungseinheit (50) mindestens ein Defektbild des Defekts (30) aufnehmbar ist.

16. System nach Anspruch 15, **dadurch gekennzeichnet, daß** von der Bilderfassungseinheit (50) ein Bilddatensatz des mindestens einen Defektbilds bereitstellbar ist und daß der Bilddatensatz zum Defektdatensatz verarbeitbar ist.

17. System nach Anspruch 16 **dadurch gekennzeichnet, daß** eine Datenverarbeitungsvorrichtung (14, 26) vorgesehen ist zum Verarbeiten des Bilddatensatzes mit der Datenverarbeitungseinrichtung (14, 26) zum Defektdatensatz.

18. System nach einem der Ansprüche 15 bis 17, **dadurch gekennzeichnet, daß** als Bilderfassungseinheit (50) mindestens ein Infrarotsensor vorgesehen ist.

19. System nach einem der voranstehenden Ansprüche, **dadurch gekennzeichnet, daß** ein Bearbeitungswerkzeug (52, 68) zum Bearbeiten des Implantatmaterials (54) vorgesehen ist und daß das Implantatmaterial (54) mit dem Bearbeitungswerkzeug (52, 68) unter Verwendung des Defektdatensatzes bearbeitbar ist.

20. System nach Anspruch 19, **dadurch gekennzeichnet, daß** das Bearbeitungswerkzeug (52, 68) ein von der Nachweisvorrichtung (16, 18) detektierbares Referenzelement (20) aufweist und daß das Implantatmaterial (54) mit dem Bearbeitungswerkzeug (52, 68) unter gleichzeitiger Detektion von Lage und Orientierung des Bearbeitungswerkzeugs (52, 68) bearbeitbar ist.

21. System nach einem der Ansprüche 19 oder 20, **dadurch gekennzeichnet, daß** das Bearbeitungswerkzeug (52) ein Markierwerkzeug umfaßt zum Übertragen des Defektdatensatzes auf das Implantatmaterial (54).

22. System nach einem der Ansprüche 19 bis 21, **dadurch gekennzeichnet, daß** das Bearbeitungswerkzeug (68) eine Schneidvorrichtung umfaßt zum Ausschneiden des Trägers (58) aus dem Implantatmaterial (54) unter Verwendung des Defektdatensatz.

23. System nach einem der Ansprüche 19 bis 22, **dadurch gekennzeichnet, daß** eine Bearbeitungsvorrichtung (72) zum Bearbeiten des Implantatmaterials (54) vorgesehen ist, daß der Defektdatensatz auf die Bearbeitungsvorrichtung (72) übertragbar ist und daß das Implantatmaterial (54) mit der Bearbeitungsvorrichtung (72) bearbeitbar ist.

24. System nach Anspruch 23, **dadurch gekennzeichnet, daß** die Bearbeitungsvorrichtung (72) das Bearbeitungswerkzeug (68) umfaßt.

25. System nach einem der voranstehenden Ansprüche, **dadurch gekennzeichnet, daß** das System (10) das Implantat (58) umfaßt.

26. Verfahren zum Präparieren eines Implantats zum Ausfüllen eines aufgrund eines Traumas und/oder Degeneration vorliegenden Defekts an einem Knochen und/oder einem Knorpel eines menschlichen oder tierischen Körpers, umfassend die Schritte:
- Bereitstellen mindestens eines Implantatmaterials,
- Bereitstellen eines Defektdatensatzes, welcher Daten zur Beschreibung insbesondere von Form, Fläche und/oder Volumen des Defekts enthält, und
- Präparieren des dem Defekt in Form und Größe entsprechenden Implantats aus dem Implantatmaterial unter Verwendung des Defektdatensatzes.

27. Verfahren nach Anspruch 26, **dadurch gekennzeichnet, daß** der Defekt ein Knorpeldefekt ist, daß das Implantat ein Knorpelersatzimplantat für die autologe Chondrocyten Transplantation (ACT) ist und daß mit dem Knorpelersatzimplantat der Knorpeldefekt ausgefüllt wird.

28. Verfahren nach Anspruch 27, **dadurch gekennzeichnet, daß** als Implantatmaterial ein für die Beimpfung mit Knorpelzellen geeignetes Trägermaterial bereitgestellt wird, daß das Knorpelersatzimplantat einen Träger für die Knorpelzellen umfaßt und daß der Träger aus dem Trägermaterial präpariert wird.

29. Verfahren nach Anspruch 28, **dadurch gekennzeichnet, daß** als Trägermaterial ein Vlies verwendet wird.

30. Verfahren nach einem der Ansprüche 28 oder 29, **dadurch gekennzeichnet, daß** der Träger nach dem Präparieren und vor dem Implantieren mit Knorpelzellen beimpft wird.

31. Verfahren nach einem der Ansprüche 28 bis 30, **dadurch gekennzeichnet, daß** vor der Implantation des Knorpelersatzimplantats Knorpelzellen aus dem menschlichen oder tierischen Körper entnommen werden, daß die entnommenen körpereigenen Knorpelzellen im Labor vermehrt werden und daß die vermehrten körpereigenen Knorpelzellen zum Beimpfen des Trägers bereitgestellt werden.

32. Verfahren nach einem der Ansprüche 28 bis 31, **dadurch gekennzeichnet, daß** aus dem Defektdatensatz das Defektvolumen des Knorpeldefekts bestimmt wird und daß ein Implantationsvolumen von Knorpelzellen bereitgestellt wird, wobei das Implantationsvolumen ausreichend ist, um den Träger für eine Implantation des Knorpelersatzimplantats ausreichend mit Knorpelzellen zu beimpfen.

33. Verfahren nach Anspruch 32, **dadurch gekennzeichnet, daß** das bereitgestellte Trägermaterial Hohlräume zur Aufnahme von Knorpelzellen aufweist, daß die Hohlräume des Trägers ein Hohlraumvolumen aufweisen und daß ein Implantationsvolumen bereitgestellt wird, das dem Hohlraumvolumen oder in etwa dem Hohlraumvolumen des Trägers entspricht.

34. Verfahren nach einem der Ansprüche 26 bis 33 **dadurch gekennzeichnet, daß** der Defektdatensatz mit einer Datenverarbeitungseinrichtung zu einem Präparierdatensatz verarbeitet wird, wobei der Präparierdatensatz Daten zur Beschreibung insbesondere von Form, Fläche und/oder Volumen des zu präparierenden Implantats enthält.

35. Verfahren nach einem der Ansprüche 26 bis 34, **dadurch gekennzeichnet, daß** zum Bestimmen des Defektdatensatzes ein minimalinvasiver Zugang in den menschlichen oder tierischen Körper eröffnet wird.

36. Verfahren nach einem der Ansprüche 26 bis 35, **dadurch gekennzeichnet, daß** vor dem Bestimmen des Defektdatensatzes ein Rand des Defekts präpariert wird.

37. Verfahren nach einem der Ansprüche 26 bis 36, **dadurch gekennzeichnet, daß** ein Meßinstrument bereitgestellt wird, an welchem ein von einer Nachweisvorrichtung eines Navigationssystems detektierbares Referenzelement anordenbar ist zum Bestimmen von Lage und Orientierung des Meßinstruments im Raum, und daß mit dem Meßinstrument der Defekt zum Bestimmen des Defektdatensatzes vermessen und gleichzeitig die Lage und die Orientierung des Meßinstruments im Raum bestimmt wird.

38. Verfahren nach Anspruch 37, **dadurch gekennzeichnet, daß** das Meßinstrument durch den minimalinvasiven Zugang in den menschlichen oder tierischen Körper eingeführt wird.

39. Verfahren nach einem der Ansprüche 37 oder 38, **dadurch gekennzeichnet, daß** das Meßinstrument eine Tastspitze aufweist und daß zum Ausmessen des Defekts ein Rand oder eine Begrenzung des Defekts mit der Tastspitze palpiert wird.

40. Verfahren nach einem der Ansprüche 26 bis 39, **dadurch gekennzeichnet, daß** der Defekt berührungslos ausgemessen wird.

41. Verfahren nach einem der Ansprüche 26 bis 40, **dadurch gekennzeichnet, daß** das Meßinstrument eine optische Bilderfassungseinheit aufweist und daß mit der Bilderfassungseinheit mindestens ein Defektbild des Defekts aufgenommen wird.

42. Verfahren nach Anspruch 41, **dadurch gekennzeichnet, daß** von der Bilderfassungseinheit ein Bilddatensatz des mindestens einen Defektbilds bereitgestellt wird und daß der Bilddatensatz zum Defektdatensatz verarbeitet wird.

43. Verfahren nach Anspruch 42 **dadurch gekennzeichnet, daß** der Bilddatensatz mit einer Datenverarbeitungsvorrichtung zum Defektdatensatz verarbeitet wird.

44. Verfahren nach einem der Ansprüche 41 bis 43, **dadurch gekennzeichnet, daß** als Bilderfassungseinheit Infrarotsensoren verwendet werden.

45. Verfahren nach einem der Ansprüche 26 bis 44, **dadurch gekennzeichnet, daß** ein Bearbeitungswerkzeug zum Bearbeiten des Implantatmaterials bereitgestellt wird und daß das Implantatmaterial mit dem Bearbeitungswerkzeug unter Verwendung des Defektdatensatzes bearbeitet wird.

46. Verfahren nach Anspruch 45, **dadurch gekennzeichnet, daß** das Bearbeitungswerkzeug ein von der Nachweisvorrichtung detektierbares Referenzelement aufweist und daß das Implantatmaterial mit dem Bearbeitungswerkzeug unter gleichzeitiger Detektion von Lage und Orientierung des Bearbeitungswerkzeugs bearbeitet wird.

47. Verfahren nach einem der Ansprüche 45 oder 46, **dadurch gekennzeichnet, daß** als Bearbeitungswerkzeug ein Markierstift zum Übertragen des Defektdatensatzes auf das Implantatmaterial bereitgestellt wird.

48. Verfahren nach einem der Ansprüche 45 bis 47, **dadurch gekennzeichnet, daß** als Bearbeitungswerkzeug eine Schneidvorrichtung zum Ausschneiden des Trägers aus dem Implantatmaterial unter Verwendung des Defektdatensatz bereitgestellt wird.

49. Verfahren nach einem der Ansprüche 45 bis 48, **dadurch gekennzeichnet, daß** eine Bearbeitungsvorrichtung zum Bearbeiten des Implantatmaterials bereitgestellt wird, daß der Defektdatensatz auf die Bearbeitungsvorrichtung übertragen wird und daß das Implantatmaterial mit der Bearbeitungsvorrichtung bearbeitet wird.

50. Verfahren nach Anspruch 49, **dadurch gekennzeichnet, daß** die bereitgestellte Bearbeitungsvorrichtung das Bearbeitungswerkzeug umfaßt.
